# FASCICULE DE BREVET EUROPEEN

(11) **EP 3 720 527 B1**
(45) Date de publication et mention de la délivrance du brevet: **15.09.2021**
(21) Numéro de dépôt: 18811046.4
(22) Date de dépôt: 05.12.2018
(51) Int. Cl.: A61M 5/32

(54) **SYSTÈME DE SÉCURITÉ APRÈS USAGE POUR UNE SERINGUE À AIGUILLE COLLÉE ET SERINGUE ÉQUIPÉE D'UN TEL SYSTÈME**
NACHGEBRAUCHSSICHERHEITSSYSTEM FÜR EINE SPRITZE MIT GEKLEBTER NADEL UND SPRITZE MIT EINEM SOLCHEN SYSTEM
AFTER-USE SAFETY SYSTEM FOR A SYRINGE WITH BONDED NEEDLE AND SYRINGE EQUIPPED WITH SUCH A SYSTEM

(30) Priorité: 06.12.2017 FR 1761719
(43) Date de publication de la demande: 14.10.2020
(73) Titulaire: Biocorp Production, 63500 Issoire (FR)
(72) Inventeur: ANEAS, Daniel, 63200 Menetrol (FR)
(74) Mandataire: Lavoix
(86) Numéro de dépôt international: PCT/EP2018/083605
(87) Numéro de publication internationale: WO 2019/110644

(56) Documents cités:
- EP-A1- 3 106 191
- WO-A1-2013/134465
- WO-A1-2016/207196
- WO-A2-2016/120185
- US-A1- 2005 267 416
- US-A1- 2008 167 611

## Description

La présente invention concerne un système de sécurité après usage pour une seringue à aiguille collée.

Dans le domaine médical, un système de sécurité après usage est un système permettant de protéger (ou recouvrir) l'aiguille d'une seringue à la fin de l'injection. Cela permet d'éviter de se blesser avec l'aiguille lorsque celle-ci est retirée du corps du patient et permet de lutter contre la transmission de maladies, comme le VIH.

Typiquement, les figures 1 à 5, extraites de la publication WO 2016/120185 A1, montrent un exemple de système de sécurité après usage. Ce système comprend un fourreau 18 qui définit un axe central X1 selon lequel il est mobile en translation et qui comprend deux ouvertures radiale 180 formant chacune un chemin de guidage pour un pion 160. Les deux pions 160 appartiennent à un bloqueur 16b, qui est disposé à l'intérieur du fourreau. Le système comprend également un ressort de rappel 20, configuré pour charger élastiquement le fourreau 18 vers l'avant par rapport au bloqueur 16b. Chaque chemin de guidage 180 est globalement en forme de Y asymétrique, avec les branches du Y qui s'étendent vers l'arrière. Les branches du Y sont référencées 180a et 180c, alors que sa portion centrale est référencée 180b. Cette portion centrale 180b est une portion droite, c'est-à-dire un couloir.

Avantageusement, chaque chemin de guidage 180 comprend également, à une extrémité, un tronçon coudé formant un logement 180d, à l'intérieur duquel le pion correspondant 160 est reçu lorsque le fourreau 18 est forcé vers l'arrière, de façon à bloquer le déplacement du fourreau vers l'arrière après usage de la seringue. On parle de moyens de verrouillage en position avancée.

Lorsque la seringue est amenée contre l'épiderme d'un patient, la pression exercée par le fourreau sur la peau entraine le recul du fourreau 18. Le ressort 20 est alors comprimé et l'aiguille 10 pénètre dans l'épiderme. Les pions 160 se déplacent de la branche 180a jusque dans la portion centrale 180b. Lorsque l'aiguille est complètement enfoncée dans l'épiderme, l'utilisateur appuie sur la tige 4 de la seringue pour injecter le principe actif contenu à l'intérieur de la seringue 1 dans le corps du patient.

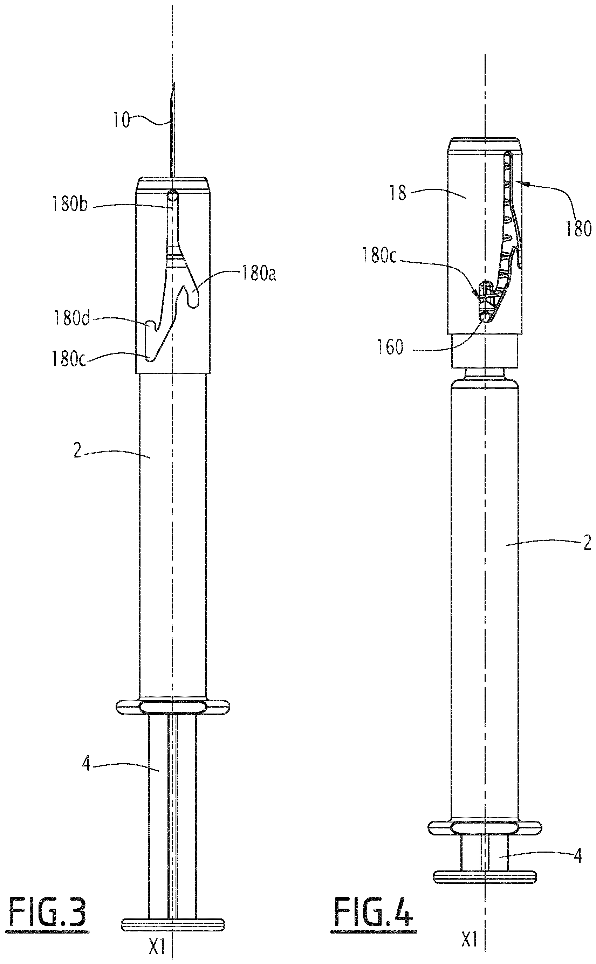

Lorsque l'utilisateur retire la seringue du corps du patient, le fourreau 18 est rappelé élastiquement en position avancée par le ressort 20. Le fourreau revient alors en recouvrement de l'aiguille et les pions coulissent dans le couloir 180b des ouvertures en direction de la branche 180c. La seringue se trouve alors dans la configuration de la figure 4, qui est une configuration de fin d'injection.

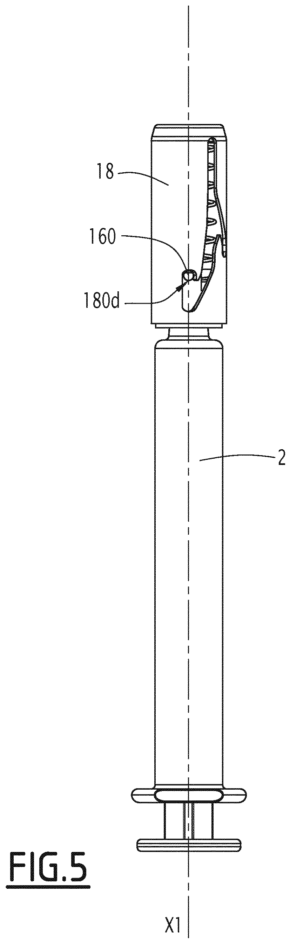

Si, après usage de la seringue, un utilisateur maladroit appuie sur le fourreau, c'est-à-dire tente de déplacer le fourreau vers l'arrière, alors les pions 160 se déplacent, comme visible à la figure 5, dans le logement 180d et le déplacement du fourreau vers l'arrière est bloqué. Cela constitue une sécurité supplémentaire puisque l'aiguille ne peut plus être découverte en fin d'injection.

Néanmoins, le fourreau est généralement réalisé en matière plastique, avec une épaisseur de paroi relativement faible, si bien que le fourreau a une certaine propension à se déformer lorsque l'on force dessus vers l'arrière, c'est-à-dire lorsque l'on applique un effort axial à une extrémité longitudinale du fourreau. Cette déformation est d'ailleurs également favorisée par la présence des chemins de guidage pratiqués dans le fourreau, qui diminuent la résistance mécanique du fourreau.

Ainsi, on constate en pratique que lorsque l'effort axial atteint approximativement 20N, le fourreau a tendance à fléchir et s'ovaliser. Les pions entrent alors à l'intérieur du fourreau et sortent de leur chemin de guidage : le système est déverrouillé.

C'est à ces inconvénients qu'entend plus particulièrement remédier l'invention en proposant un nouveau système de sécurité après usage, qui est plus résistant à une tentative de déverrouillage en force.

Une solution évidente pour améliorer la résistance de l'ensemble à une tentative de déverrouillage en force consiste à augmenter la taille des pions et/ou du fourreau. En revanche, le système ne serait plus aussi compact radialement, ce qui poserait des problèmes lors de la fabrication des seringues. Typiquement, le système ne serait plus suffisamment compact pour pouvoir pénétrer dans les logements des supports utilisés de façon standard dans le domaine (Voir à ce sujet la problématique exposée dans la publication WO 2016/120185 A1, dont le contenu est incorporé ici par référence).

À cet effet l'invention concerne un système de sécurité après usage pour une seringue, ce système étant conforme à la revendication 1.

Selon des aspects avantageux, mais non obligatoires de l'invention, le système peut comprend une ou plusieurs des caractéristiques suivantes, prises dans toute combinaison techniquement admissible :
- le moyen d'arrêt radial fait partie du pion.
- le moyen d'arrêt radial est une partie d'extrémité radiale du pion, qui est disposée à l'opposé par rapport à la base du pion.
- ladite partie du fourreau comprend deux faces obliques complémentaires de celles du pion, les faces obliques de ladite partie du fourreau coopérant avec les faces obliques du pion lorsque le fourreau est forcé vers l'arrière après l'usage de la seringue.
- le moyen d'arrêt radial comprend une surface d'arrêt coopérant avec une surface complémentaire du fourreau lorsque le fourreau est forcé vers l'arrière après usage de la seringue.
- la surface d'arrêt est non-perpendiculaire à l'axe central, de préférence inclinée par rapport à l'axe central.
- la surface d'arrêt comprend une normale dirigée vers l'intérieur du système.

L'invention concerne également une seringue, équipée d'un système de sécurité après usage tel que défini précédemment.

L'invention et d'autres avantages de celle-ci apparaitront plus clairement à la lumière de la description qui va suivre d'un mode de réalisation d'un système de sécurité après usage conforme à son principe, donnée uniquement à titre d'exemple et faite en référence aux dessins dans lesquels :
- Les figures 1 à 5 sont des vues d'une seringue équipée d'un système de sécurité après usage de l'art antérieur, et montrent notamment les différentes configurations du système lorsqu'il est monté sur une seringue et que la seringue est utilisée pour injecter un principe actif à l'intérieur du corps d'un patient ;
- La figure 6 est une vue de côté du système de sécurité après usage selon l'invention, lequel comprend un fourreau et un bloqueur disposé à l'intérieur du fourreau ;
- La figure 7 est une vue à plus grande échelle de l'encadré VII de la figure 6 ;
- La figure 8 est une vue en perspective du bloqueur appartenant au système de sécurité selon l'invention ;
- La figure 9 est une vue à plus grande échelle de l'encadré IX de la figure 8 ;
- La figure 10 est une coupe du système de sécurité après usage, alors monté sur une seringue, le plan de coupe passant, dans cette vue, par la base d'un pion du bloqueur ;
- La figure 11 est une vue à plus grande échelle de l'encadré XI de la figure 10 ;
- La figure 12 est une coupe longitudinale du système de sécurité après usage ; et
- La figure 13 est une vue à plus grande échelle de l'encadré XIII de la figure 12.

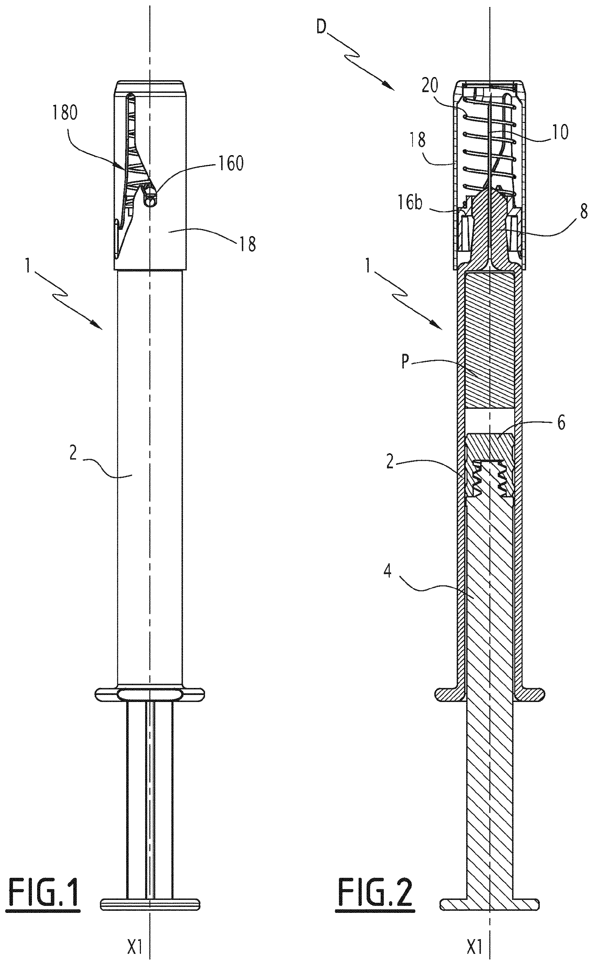

Les figures 1 et 2 montrent une seringue 1 à aiguille collée. La seringue 1 s'étend selon un axe longitudinal X1 et comprend un corps de seringue 2 contenant un principe actif P. Le corps de seringue 2 comporte une extrémité longitudinale 8 formant le nez de la seringue. La seringue comprend également une aiguille hypodermique 10 montée à demeure (inamovible). L'aiguille 10 est collée à l'intérieur d'un trou traversant de l'extrémité 8 du corps.

La seringue 1 comprend aussi un piston 6 et une tige de piston 4. Avantageusement, la tige de piston 4 et le piston 6 sont vissés l'un avec l'autre. La tige de piston 4 sert classiquement à pousser le principe actif P contenu à l'intérieur du corps de seringue 2 à l'intérieur de l'aiguille 10, laquelle est bien entendu creuse. La tige de piston 4 est mobile en translation à l'intérieur du corps 2. Elle peut être manœuvrée à une extrémité longitudinale de la seringue, notamment à l'extrémité opposée au nez de la seringue.

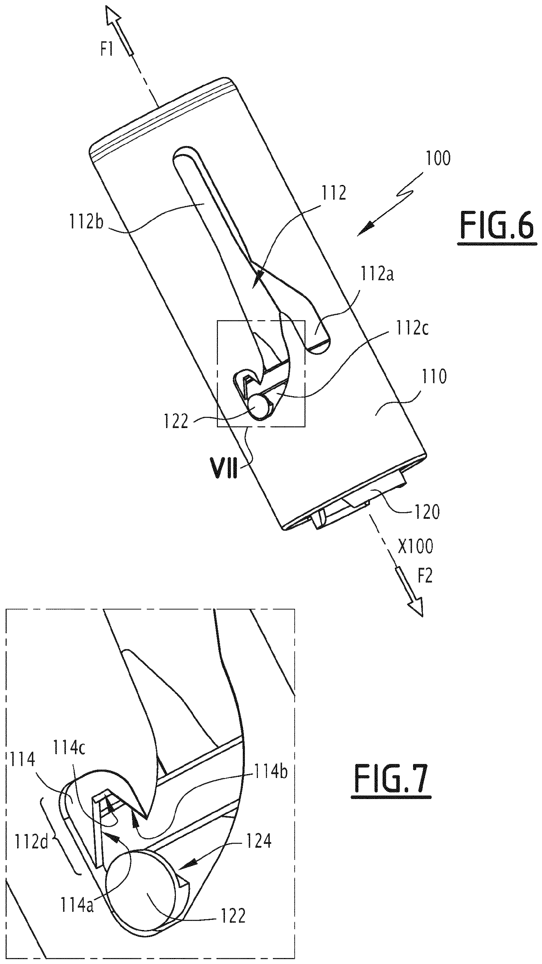

Un système de sécurité après usage 100, représenté sur les figures 6 à 11, est configuré pour être monté sur une seringue à aiguille collée, telle que celle représentée aux figures 1 à 5 par exemple. Ce système 100 a pour fonction principale de recouvrir l'aiguille de la seringue après usage, c'est-à-dire à la fin de l'injection, afin de prévenir les piqûres accidentelles avec la seringue usagée. Ce système a notamment pour vocation de protéger le personnel qui manipule les seringues après usage.

Ce système de sécurité après usage 100 comprend un fourreau 110, qui définit un axe central X100 selon lequel il est mobile en translation et qui comprend au moins une, de préférence deux ouvertures radiales 112 formant respectivement deux chemins de guidage pour des pions 122.

Classiquement, chaque chemin de guidage 112 est globalement en forme de Y asymétrique, avec les branches du Y qui s'étendent vers l'arrière. Les branches du Y sont référencées 112a et 112c, alors que sa portion centrale est référencée 112b. Cette portion centrale 112b est une portion droite, c'est-à-dire un couloir.

De manière astucieuse, chaque chemin de guidage 112 comprend, à une extrémité, notamment à l'extrémité libre de la branche 112c un tronçon coudé formant un logement 112d, particulièrement visible à la figure 7, à l'intérieur duquel un pion 122 est reçu lorsque le fourreau 110 est forcé vers l'arrière après usage de la seringue. Ce logement 112d forme en fait un moyen de verrouillage du fourreau en position de recouvrement de l'aiguille (position avancée).

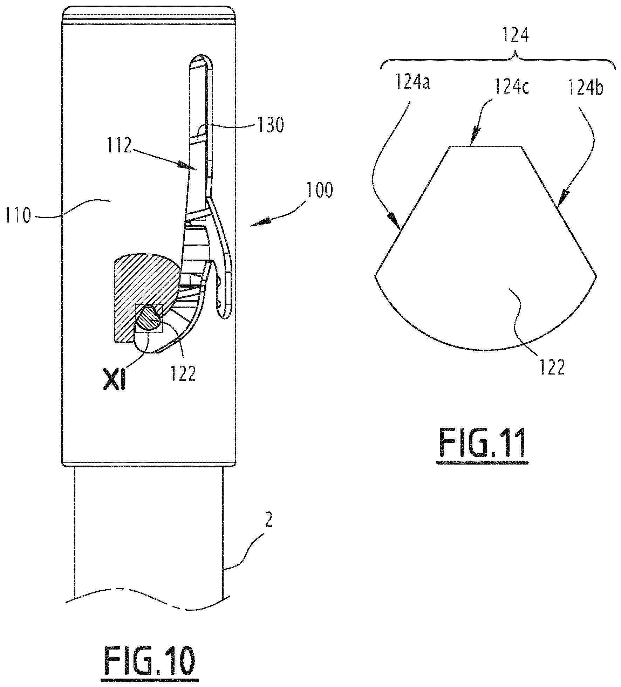

Le système 100 comprend également ce qu'on appelle un bloqueur 120, qui est disposé à l'intérieur du fourreau 110 et qui comprend les pions 122 coopérant avec le fourreau 110. Le système 100 comprend enfin un ressort de rappel 130, visible notamment à la figure 10 et configuré pour charger élastiquement le fourreau 110 vers l'avant par rapport au bloqueur 120.

La direction avant est représentée à la figure 6 par une flèche F1, la flèche F2 représentant la direction arrière. Les directions avant et arrière sont des directions opposées parallèles à l'axe central X100 du fourreau 110.

Le fourreau 110 est une pièce de géométrie tubulaire centrée sur l'axe X100. Le fourreau 110 est de préférence réalisé en matière plastique, notamment suivant un procédé de moulage par injection.

Le bloqueur 120 prend la forme d'une pièce globalement cylindrique, creuse, et centrée également sur l'axe X100. On peut donc dire que le bloqueur 120 est disposé coaxialement à l'intérieur du fourreau 110. Dans l'exemple, les pions 122 sont au nombre de deux et s'étendent radialement vers l'extérieur, ici de manière diamétralement opposée l'un par rapport à l'autre. Plus précisément, les pions 122 font saillie radialement par rapport au reste du corps du bloqueur 120.

Bien évidemment, le nombre de pions 122 n'est pas limitatif. On pourrait, en variante, avoir un, trois ou encore plus de pions 122. Toutefois, il y a un chemin de guidage pour chaque pion.

Avantageusement, chaque pion 122 ne dépasse pas radialement par rapport à une surface radiale externe S110 du fourreau 110. Ainsi, le diamètre maximal du bloqueur 120 est inférieur au diamètre maximal du fourreau 110. Ceci rend le système de sécurité après usage 100 compact radialement.

En pratique, le bloqueur 120 est configuré pour être fixé, éventuellement avec une possibilité de rotation, autour de l'extrémité du corps de seringue. Par exemple, le bloqueur 120 peut comprendre, de façon connue en soi, des pattes élastiques d'accrochage (non représentées).

Le ressort 130 est un ressort spiral classique, qui est disposé à l'intérieur du fourreau 110. Le ressort 130 comprend une première extrémité au contact du bloqueur 120 et une deuxième extrémité au contact d'un épaulement radial interne du fourreau 110 (voir pour exemple la figure 2). Cet épaulement radial interne se retrouve à une extrémité longitudinale du fourreau, notamment à l'extrémité avant.

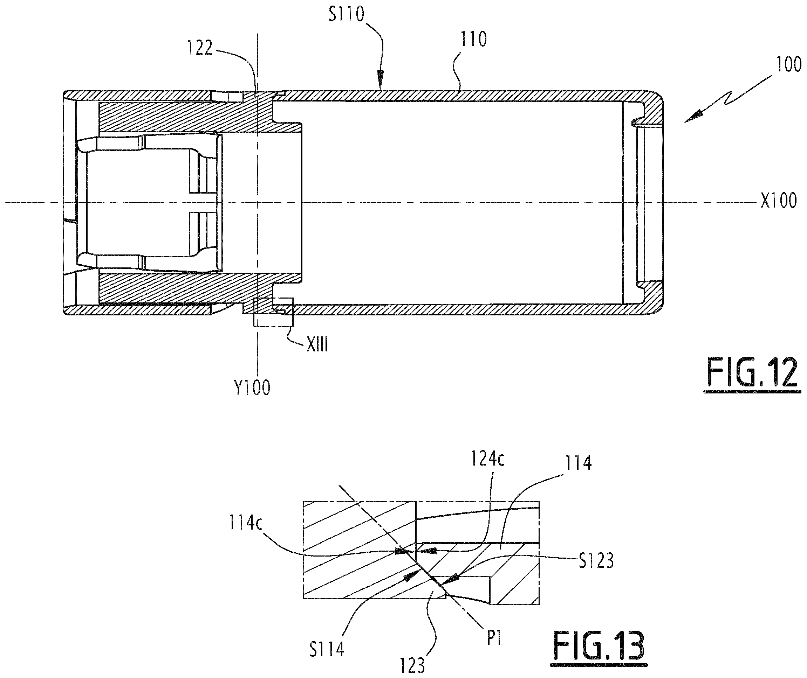

Comme visible plus particulièrement aux figures 12 et 13, le bloqueur 120 comprend un moyen d'arrêt radial 123 pour bloquer le fourreau 110 selon un axe Y100 radial à l'axe central X100 lorsque le fourreau 110 est forcé vers l'arrière après usage de la seringue.

De préférence, le moyen d'arrêt radial 123 fait partie du ou des pions 122. Dans l'exemple, chaque pion 122 comprend un moyen d'arrêt radial 123.

Avantageusement, chaque moyen d'arrêt radial 123 est une partie d'extrémité radiale du pion, qui est disposée à l'opposé par rapport à la base du pion qui, elle, est solidaire du reste du bloqueur 120.

Dans le mode de réalisation des figures, chaque pion 122 comprend un évidement 124 en partie recouvert radialement par le moyen d'arrêt radial 123. En d'autres termes, le moyen d'arrêt radial 123 cache l'évidement 124 lorsque l'on regarde le pion 122 par le haut. Cela signifie que le moyen d'arrêt radial 123 est disposé au-dessus de l'évidement 124. L'évidement 124 est dimensionné pour recevoir une partie 114 du fourreau lorsque le fourreau 110 est forcé vers l'arrière après usage de la seringue.

Ici, la partie 114 est formée à l'intérieur du logement 112d formé à l'extrémité libre de la branche 112c de l'ouverture 112.

Comme visible à la figure 7, cette partie 114 du fourreau 110 comprend avantageusement deux faces obliques 114a et 114b qui convergent l'une en direction de l'autre, de préférence de façon symétrique, vers l'avant.

Dans l'exemple, la partie 114 du fourreau comprend également une face 114c de jonction entre les deux faces obliques 114a et 114b. De préférence, cette face de jonction 114c est plane, mais on pourrait aussi imaginer, en variante, qu'elle soit de forme convexe, c'est-à-dire bombée.

Dans l'exemple, l'évidement 124 s'étend sur une partie uniquement de la circonférence du pion 122. L'évidement 124 est agencé à la base du pion 122.

Avantageusement, les parois de l'évidement 124 comprennent deux faces obliques 124a et 124b qui convergent l'une en direction de l'autre, de préférence de façon symétrique, vers l'avant.

Les deux faces obliques 124a et 124b sont complémentaires de celles de la paroi 114, les faces obliques 114a et 114b de la partie 114 coopérant avec les faces obliques 124a et 124b du pion 122 lorsque le fourreau 110 est forcé vers l'arrière après l'usage de la seringue.

Les parois de l'évidement comprennent une face 124c de jonction entre les deux faces obliques 124a et 124b. De préférence, cette face de jonction 124c est plane, mais on pourrait aussi imaginer, en variante, qu'elle soit de forme concave, c'est-à-dire creuse.

Avantageusement, et comme visible à la figure 13, chaque moyen d'arrêt radial 123 comporte une surface d'arrêt S123 destinée à buter contre une surface S114 du fourreau 110 lorsque le fourreau 110 est forcé vers l'arrière après usage de la seringue. Plus précisément, cette surface S114 est délimitée par la partie 114 du fourreau définie précédemment.

Dans l'exemple, les surfaces S114 et S123, qui coopèrent de façon complémentaire l'une avec l'autre, sont des surfaces inclinées par rapport à l'axe central X100 du système 100. Ainsi, et comme visible à la figure 13, le plan P1 de contact entre les deux surfaces S114 et S123 est un plan oblique (c'est-à-dire incliné) par rapport à un plan longitudinal ou transversal du système 100.

En variante non représentée, les surfaces S114 et S123 pourraient être des surfaces radiales, c'est-à-dire des surfaces perpendiculaires à l'axe radial Y100 du système. Plus généralement, les surfaces S114 et S123 ne sont chacune pas perpendiculaires à l'axe central X100.

Avantageusement, la normale de la surface S114 est dirigée vers l'extérieur du système 100, alors que la normale de la surface S123 est dirigée vers l'intérieur du système. De cette façon, lorsque le fourreau 100 est forcé vers l'arrière après usage de la seringue, les pions 122 s'opposent, grâce à la partie 123, à l'expansion radiale du fourreau (ovalisation).

En dessous d'un certain seuil d'effort axial, typiquement de l'ordre de 40N, chaque pion 122 est bloqué en butée contre la partie 114 du fourreau 110 lorsque le fourreau est forcé vers l'arrière après usage de la seringue 1.

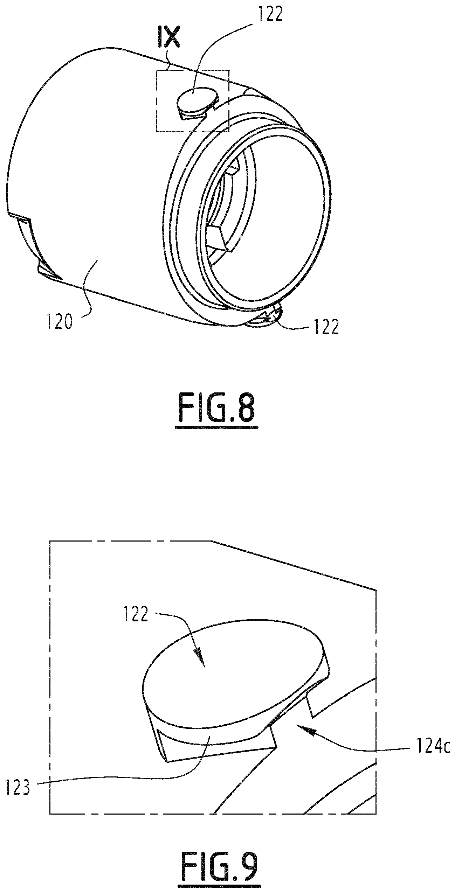

En revanche, au-dessus de ce seuil, le moyen d'arrêt radial 123 est arraché au contact du fourreau 11. Ainsi, on peut dire que le moyen d'arrêt radial est, dans un mode de réalisation préférentiel, une partie arrachable du pion 122 (visible uniquement à la figure 9), qui est arrachée au contact de la partie 114 du fourreau lorsque le fourreau 110 est forcé vers l'arrière après usage de la seringue.

Dans l'exemple, chaque pion 122 est dimensionné de telle façon que le moyen d'arrêt radial 123 est arraché au contact du fourreau 110 dès lors que l'effort axial appliqué sur le fourreau dépasse les 40N.

Ainsi, la résistance du système à une tentative de déverrouillage en force après usage de la seringue dépend désormais de la résistance à l'arrachement du ou des pions du bloqueur, et non de la résistance du fourreau lui-même à la déformation. Dans l'exemple où le bloqueur comprend deux pions, on constate que les pions sont arrachés à partir de 40N d'effort axial, contre 20N auparavant. Cela provient du fait que la résistance mécanique du système au déverrouillage ne repose plus sur la résistance mécanique du fourreau à la déformation, et notamment à l'expansion radiale (qui est relativement faible), mais plutôt sur la résistance à l'arrachement des pions, plus élevée.

Après usage de la seringue, le système 100 se trouve dans la configuration de la figure 6. A partir de cette configuration, si on tente de reculer le fourreau 110, c'est-à-dire si on pousse le fourreau 110 dans la direction de la flèche F2, alors le ou les pions 122 prennent chacun la direction du logement 112d. Les faces 124a à 124c viennent respectivement en butée contre les faces 114a à 114c. Egalement, le moyen d'arrêt radial 123 de chaque pion 122 bloque la déformation radiale du fourreau 110, de manière localisée au niveau de chaque pion 122. Dans l'exemple, comme les pions 122 sont au nombre de deux et sont disposés de façon diamétralement opposée par rapport à l'axe central X100, les moyens d'arrêt radial 123 des deux pions exercent des efforts de résistance sensiblement alignés suivant l'axe radial Y100 et dirigés l'un en direction de l'autre.

Lorsque l'effort axial dépasse 40N environ, la partie 123 de chaque pion 122 est arrachée, et chaque pion 122, alors dépourvu de la partie 123, passe à l'intérieur du fourreau 110. Notamment, on observe une certaine ovalisation du fourreau, c'est-à-dire une expansion radiale localisée, menant le ou les pions 122 à entrer à l'intérieur du fourreau 110, c'est-à-dire à sortir de l'ouverture 112 correspondante.

On comprend donc que les pions 122 ont une certaine fragilité à la rupture, c'est-à-dire une forme propice à la rupture, de sorte que lorsque l'on force le fourreau 110 vers l'arrière après usage de la seringue, on observe la rupture du pion plutôt que la déformation du fourreau 110.

## Revendications

1. Système de sécurité après usage (100) pour une seringue (1), ce système comprenant :
- un fourreau (110), qui définit un axe central (X100) selon lequel il est mobile en translation et qui comprend une ouverture radiale (112) formant un chemin de guidage pour un pion (122),
- un bloqueur (120), qui est disposé à l'intérieur du fourreau et qui comprend le pion coopérant avec le fourreau, et
- un ressort de rappel (130), configuré pour charger élastiquement le fourreau vers l'avant par rapport au bloqueur,
**caractérisé en ce que**
- le bloqueur (120) comprend un moyen d'arrêt radial (123) pour bloquer le fourreau (110) selon un axe (Y100) radial à l'axe central (X100) lorsque le fourreau (110) est forcé vers l'arrière après usage de la seringue,
- le pion comprend un évidement (124) au moins en partie recouvert par le moyen d'arrêt radial (123) et **en ce que** cet évidement (124) est dimensionné pour recevoir une partie (114) du fourreau lorsque le fourreau est forcé vers l'arrière après usage de la seringue, et
- les parois (124a, 124b, 124c) de l'évidement (124) comprennent deux faces obliques (124a, 124b) qui convergent l'une en direction de l'autre, de préférence de façon symétrique, vers l'avant.

2. Système de sécurité après usage selon la revendication 1, **caractérisé en ce que** le moyen d'arrêt radial (123) fait partie du pion (122).

3. Système de sécurité après usage selon la revendication 2, **caractérisé en ce que** le moyen d'arrêt radial (123) est une partie d'extrémité radiale du pion, qui est disposée à l'opposé par rapport à la base du pion.

4. Système de sécurité après usage selon la revendication 1, **caractérisé en ce que** ladite partie (114) du fourreau (110) comprend deux faces obliques (114a, 114b) complémentaires de celles du pion, les faces obliques de ladite partie du fourreau coopérant avec les faces obliques (124a, 124b) du pion (122) lorsque le fourreau est forcé vers l'arrière après l'usage de la seringue.

5. Système de sécurité après usage selon l'une des revendications précédentes, **caractérisé en ce que** le moyen d'arrêt radial (123) comprend une surface d'arrêt (S123) coopérant avec une surface complémentaire (S114) du fourreau lorsque le fourreau (110) est forcé vers l'arrière après usage de la seringue.

6. Système de sécurité après usage selon la revendication 5, **caractérisé en ce que** la surface d'arrêt (S123) est non-perpendiculaire à l'axe central (X100), de préférence inclinée par rapport à l'axe central (X100).

7. Système de sécurité après usage selon la revendication 5 ou 6, **caractérisé en ce que** la surface d'arrêt (S123) comprend une normale dirigée vers l'intérieur du système.

8. Seringue (1), équipée d'un système de sécurité après usage (100) selon l'une des revendications précédentes.

## Patentansprüche

1. Sicherheitssystem (100) für eine Spritze (1) nach Gebrauch, wobei das System umfasst:
- eine Hülse (110), die eine zentrale Achse (X100) definiert, entlang der sie translatorisch beweglich ist, und die eine radiale Öffnung (112) aufweist, die eine Führungsbahn für ein Ansatzstück (122) bildet,
- ein Blockierelement (120), das innerhalb der Hülse angeordnet ist und das das mit der Hülse zusammenwirkende Ansatzstück umfasst, und
- eine Rückstellfeder (130), die ausgebildet ist, die Hülse relativ zum Blockierelement elastisch nach vorne zu belasten,
**dadurch gekennzeichnet, dass**
- das Blockierelement (120) ein radiales Anschlagmittel (123) zum Blockieren der Hülse (110) entlang einer Achse (Y100) radial zur Mittelachse (X100) umfasst, wenn die Hülse (110) nach der Verwendung der Spritze nach hinten gedrückt wird,
- das Ansatzstück eine Ausnehmung (124) aufweist, die zumindest teilweise von dem radialen Anschlagmittel (123) bedeckt ist, und dass diese Ausnehmung (124) bemessen ist, einen Teil (114) der Hülse aufzunehmen, wenn die Hülse nach der Verwendung der Spritze nach hinten gedrückt wird, und
- die Wände (124a, 124b, 124c) der Ausnehmung (124) zwei schräge Flächen (124a, 124b) umfassen, die zueinander in Vorwärtsrichtung, vorzugsweise symmetrisch, konvergieren.

2. Sicherheitssystem nach Gebrauch nach Anspruch 1, **dadurch gekennzeichnet, dass** das radiale Anschlagmittel (123) Teil des Ansatzstücks (122) ist.

3. Sicherheitssystem nach Gebrauch nach Anspruch 2, **dadurch gekennzeichnet, dass** das radiale Anschlagmittel (123) ein radialer Endabschnitt des Ansatzstücks ist, der gegenüber der Basis des Ansatzstücks angeordnet ist.

4. Sicherheitssystem nach Gebrauch nach Anspruch 1, **dadurch gekennzeichnet, dass** der Teil (114) der Hülse (110) zwei schräge Flächen (114a, 114b) aufweist, die zu denen des Ansatzstücks komplementär sind, wobei die schrägen Flächen des Teils der Hülse mit den schrägen Flächen (124a, 124b) des Ansatzstücks (122) zusammenwirken, wenn die Hülse nach dem Gebrauch der Spritze nach hinten gedrückt wird.

5. Sicherheitssystem nach Gebrauch nach einem der vorhergehenden Ansprüche, **dadurch gekennzeichnet, dass** das radiale Anschlagmittel (123) eine Anschlagfläche (S123) aufweist, die mit einer komplementären Fläche (S114) der Hülse zusammenwirkt, wenn die Hülse (110) nach dem Gebrauch der Spritze nach hinten gedrückt wird.

6. Sicherheitssystem nach Gebrauch nach Anspruch 5, **dadurch gekennzeichnet, dass** die Anschlagfläche (S123) nicht senkrecht zur Mittelachse (X100), vorzugsweise schräg zur Mittelachse (X100), verläuft.

7. Sicherheitssystem nach Gebrauch nach Anspruch 5 oder 6, **dadurch gekennzeichnet, dass** die Anschlagfläche (S123) eine zum Inneren des Systems gerichtete Normale aufweist.

8. Spritze (1), die mit einem Sicherheitssystem nach Gebrauch (100) nach einem der vorhergehenden Ansprüche ausgestattet ist.

## Claims

1. An after-use safety system (100) for a syringe (1), this system comprising:
- a barrel (110), which defines a central axis (X100) along which it can move in translation and which comprises a radial opening (112) forming a guidance path for a pin (122),
- an immobilizer (120), which is arranged inside the barrel and which comprises the pin interacting with the barrel, and
- a return spring (130), configured to elastically load the barrel forwards with respect to the immobilizer,
**characterized in that**:
- the immobilizer (120) comprises a radial stopping means (123) for immobilizing the barrel (110) along one axis (Y100) radial to the central axis (X100) when the barrel (110) is forced towards the rear after use of the syringe,
- the pin comprises a recess (124) at least partially covered by the radial stopping means (123) and **in that** this recess (124) is dimensioned to receive a portion (114) of the barrel when the barrel is forced toward the rear after use of the syringe, and
- the walls (124a, 124b, 124c) of the recess (124) comprise two oblique faces (124a, 124b) that converge one toward the other, preferably symmetrically, in the forward direction.

2. The after-use safety system according to claim 1, **characterized in that** the radial stopping means (123) is part of the pin (122).

3. The after-use safety system according to claim 2, **characterized in that** the radial stopping means (123) is a radial end part of the pin, which is arranged opposite the base of the pin.

4. The after-use safety system according to claim 1, **characterized in that** said portion (114) of the barrel (110) comprises two oblique faces (114a, 114b) complementary to those of the pin, the oblique faces of said part of the barrel cooperating with the oblique faces (124a, 124b) of the pin (122) when the barrel is forced toward the rear after the use of the syringe.

5. The after-use safety system according to any one of the preceding claims, **characterized in that** the radial stopping means (123) comprises a stopping surface (S123) interacting with a complementary surface (S114) of the barrel when the barrel (110) is forced toward the rear after use of the syringe.

6. The after-use safety system according to claim 5, **characterized in that** the stopping surface (S123) is non-perpendicular to the central axis (X100), preferably inclined relative to the central axis (X100).

7. The after-use safety system according to any one of claims 5 or 6, **characterized in that** the stopping surface (S123) comprises a normal oriented toward the inside of the system.

8. A syringe (1), equipped with an after-use safety system (100) according to any one of the preceding claims.
